Europäisches Patentamt

European Patent Office

Office européen des brevets

(11) Publication number: **0 061 862
B1**

(12) **EUROPEAN PATENT SPECIFICATION**

(45) Date of publication of patent specification: **25.06.86**

(51) Int. Cl.⁴: **C 12 P 19/24,** C 12 N 9/92,
C 12 N 9/96

(21) Application number: **82301334.7**

(22) Date of filing: **16.03.82**

(54) Method for isomerizing glucose to fructose.

(30) Priority: **19.03.81 JP 39930/81**

(43) Date of publication of application:
**06.10.82 Bulletin 82/40**

(45) Publication of the grant of the patent:
**25.06.86 Bulletin 86/26**

(84) Designated Contracting States:
**DE FR GB IT**

(56) References cited:
**FR-A-2 180 005
FR-A-2 245 765
FR-A-2 276 382
FR-A-2 286 669
FR-A-2 401 170
GB-A-2 001 654
US-A-4 113 565
US-A-4 246 350**

(73) Proprietor: **TORAY INDUSTRIES, INC.
2, Nihonbashi-Muromachi 2-chome Chuo-ku
Tokyo 103 (JP)**

(72) Inventor: **Yoshioka, Toshio
1-44, Ichiriyama 2-chome
Otsu-shi Shiga-ken (JP)**
Inventor: **Teramoto, Kazuo
25-8, Nango 2-chome
Otsu-shi Shiga-ken (JP)**
Inventor: **Shimamura, Masaharu
1-8075, Nionohama 2-chome
Otsu-shi Shiga-ken (JP)**

(74) Representative: **Ellis, John Clifford Holgate et al
MEWBURN ELLIS & CO. 2/3 Cursitor Street
London EC4A 1BQ (GB)**

The file contains technical information
submitted after the application was filed and
not included in this specification

Courier Press, Leamington Spa, England.

## Description

This invention relates to an enzyme reaction method using an enzyme deactivation inhibitor. Enzymes are widely utilized as specific catalysts for a variety of industrial treatments and processes. Among such enzymes, glucose isomerase is of especially high utility. Being capable of reversible interconversion of glucose and fructose, this enzyme is generally utilized from the industrial production of fructose-containing syrup by isomerizing glucose. The enzymes such as glucose isomerase have been generally used by immobilizing the enzyme itself or the enzyme-containing fungus body according to a known insolubilization method such as a conjunction method or an inclusion method for the purpose of repeated use of the enzyme. Therefore, it is a matter of great importance for the enhancement of productivity to prevent deactivation of the enzymne in the isomerization reaction step to prolong its effective life. As a means for preventing deactivation of glucose isomerase, there has been proposed a method according to which a soluble iron salt is dissolved in the substrate solution for the isomerization reaction to let the iron ions coexist with the magnesium ions in the substrate (US Patent No. 4 008 124 issued February 15, 1977). According to this method, however, there is caused colouration or sedimentation of the substrate due to addition of iron ions and also removal thereof after termination of the reaction is troublesome. Further, no satisfactory effect is provided in case of using industrial refined glucose as substrate.

In GB—A—2 001 654 there is described an enzymatic composition of isomerizating glucose to fructose in the form of small plates or filaments which comprise microorganism cells containing glucose isomerase, a cellulose ester surrounding the cells and a filler, which may be an exchange resin saturated with $Mg^{++}$ ions, or both $Mg^{++}$ and $Co^{++}$ ions. GB—A—1 596 662 (=FR—A—2 401 170), starting from the observation that iron can activate glucose isomerase enzymes, proposes to incorporate a non-toxic water-soluble iron salt in a cell mass form of glucose isomerase.

The object of the present invention is to provide an improved method of isomerizing a glucose-containing substrate solution to a fructose-containing syrup using a glucose isomerase.

The present inventors have found that quite surprisingly, the lifetime of glucose isomerase can be drastically prolonged if the glucose-containing substrate solution is brought into contact with a synthetic organic ion exchanger to which ions of iron are bonded before the substrate is contacted with the glucose isomerase.

The invention is described in detail hereinbelow.

Regarding first the ion exchanges, there may be employed known organic ion exchangers prepared by introducing an ion exchange group to a synthetic organic polymner such as styrene, phenol, vinyl alcohol, acrylic and other like types of polymers. The synthetic organic ion exchangers are chosen because of a high rate of bonding with said iron ions, low susceptibility to the attack of microorganisms and high physical strength. Particularly preferred are styrene-based synthetic organic ion exchangers because of their high chemical and heat resistance.

As the ion exchange group, there may be used anion exchange groups such as an amino group, cation exchange groups such as a carboxylic acid group, phosphoric acid group, sulphonic acid group, etc., and other known chelate groups capable of forming coordinate bonds with said iron ions such as a hydroxyl group, carboxyl group, polycarboxylic acid group, aldehyde group, polyamino group, amino carboxylic acid group, peptide group, β-diketone group, etc., and these ion exchange groups may be used in combination. Among them, the amino group, carboxylic acid group, phosphoric acid group, sulphonic acid group, polyamino group and amino carboxylic acid group are preferred as they are easily bonded to said iron ions and can also give a high deactivation inhibitory effect. Particularly preferred are polyamino groups such as $-NH(C_2H_4NH)_nH$ (where n is a number of 1 or greater) and $-NH(CH_2)_mNH_2$ (where m is a number of 3 to 12) and amino carboxylic acid groups such as an iminodiacetic acid group and polyaminopolyacetic acid group because of their high stability of bonding to said iron ions and high deactivation preventive effect. Introduction of such ion exchange groups may be accomplished by any known suitable method. The carrier to which said iron ions are bonded is usually of an insoluble type, but it is possible to use a soluble type carrier provided it is gelled after bonding to said metal salt.

Iron has been chosen as the metal to provide the ions on the synthetic organic ion exchanger as it can give an extremely high effect and involves no problem with food contamination.

The enzyme deactivation inhibitor according to this invention is produced by reacting an iron salt with said carrier so that said iron ions are bonded to the carrier. The iron salt used in this invention may be selected from acid salts and complex salts such as halogen salts, sulphates, acetates, etc., and easily soluble acid salts are preferably used. The reaction between said iron salt and said carrier can be accomplished by passing an iron salt solution through a column packed with said carrier. The reaction is usually conducted by using 0.01 to 2 M/l or an iron salt solution at room temperature for a period of from 1 minute to 40 hours. After completion of the reaction, the reaction mixture is washed wtih water or various types of buffer solution, such a citrate buffer solution, Veronal buffer solution, acetate buffer solution, phosphate buffer solution, tris buffer solution, glycine buffer solution, Menzel buffer solution, etc. to remove unreacted salts.

In the enzyme deactivation inhibitor used in this invention, iron is preferably contained in an amount of at least 0.01 meq/g, more preferably more than 0.1 meq/g, and particularly preferably within the range of from 0.5 to 2.5 meq/g. Any small content of said metal results in an unsatisfactory effect of the inhibitor. Although no specific restriction is imposed on the upper limit for the iron content, it is hardly possible and impractical to effect bonding at a rate exceeding 2.5 meq/g.

The enzyme deactivation inhibitor is usually used in the form of powder, fine grains, resinous grains, fibres, hollow fibres, film, paper, etc. Among such forms, resinous grain and fibres are desirable as they are easy to treat. Especially, macro-reticular or porous resinous grain and fibres are preferred because of the large active surface area. In the case of fibres, the core-sheath type composite fibres using various known types of fibre-forming polymers such as polyamide, polyester, poly-α-olefin, polyacrylonitrile or copolymers thereof as core component, the multiple core type composite fibres including dope blended fibres are favoured because of high mechanical strength, and among core-sheath type composite fibres the multiple core type composite fibres and simple mixed fibres are most preferred because of excellent durability. Said product may also be used in other known suitable forms such as knitting, braid, felt, etc. It is possible to use two or more forms of the enzyme deactivation inhibitor in admixture or in combination.

When the deactivation inhibitor has lost most of its efficiacy after long-time use, it may be regenerated by treating it with a water-soluble salt, a mineral acid, an alkaline solution or a mixed solution of a combination thereof according to the type of the carrier used and again reacted with an iron salt to produce the enzyme deactivation inhibitor.

As for the enzyme reaction method according to this invention, the glucose containing substrate solution is previously contacted with said enzyme deactivation inhibitor and then the enzyme reaction is carried out. The enzyme reaction may be performed in the presence of further enzyme deactivation inhibitor. In the latter case, the glucose-containing substrate solution may be contacted with the enzymne deactivation inhibitor at any stage preceding the enzyme reaction, but it is most effective and hence most desirable to effect said contact just before commencing the enzyme reaction.

Various methods may be employed by contacting the glucose-containing substrate solution with the enzyme deactivation inhibitor, such as a batch method in which the inhibitor is added to the substrate solution and stirred, a fixed bed method in which the substrate solution is passed through layers filled with the inhibitor to such a density as to allow easy passage of the solution according to the form of use, and a continuous method, but the fixed bed method is particularly preferred as it allows contact of a large volume of substrate solution with ease. The glucose concentration in the substrate solution is usually about 1 to 70% by weight. If it is too low, the throughput rate increases, while if it is too high, passage of the solution is retarded. Thus, the glucose concentration should preferably be within the range of about 5 to 60% by weight. The contact temperature is usually about 10 to 100°C. Too low a contact temperature causes an increased viscosity of the solution to worsen its fluidity and necessitate a longer time of contact, while too high a contact temperature may cause decomposition of denaturing of the glucose and/or the substrate, so that the preferred range of contact temperature is from about 20 to 80°C. The time of contact between the enzyme deactivation inhibitor and the glucose-containing substrate solution should be suitably determined according to the metal content, substrate solution concentration, contact temperature and other factors so as to provide the maximum enzyme deactivation inhibitory effect, but usually the contact time is within the range of about 0.1 to 1,000 ml/g·hr, preferably 1 to 500 ml/g·hr. If the contact time is outside this range, there may not be provided a satisfactory enzyme deactivation inhibitory effect and also denaturing of glucose or substrate may be caused. The enzyme reaction is preferably carried out at a known substrate concentration, temperature and pH pertinent to the isomerase or insolubilized isomerase to be treated.

The enzyme reaction using a glucose-containing substrate solution according to this invention may be accomplished in various ways. 30 to 60% by weight of a glucose solution (pH 6.5—9.0) containing 0.1—20 mM/1 of magnesium ions is passed through a single or several fixed beds composed of first-stage layers packed with said enzyme deactivation inhibitor to a required density according to the type thereof and second-stage layers of immobilized glucose isomerase at a temperature of 55—70°C and with a contact time selected to provide the desired isomerization rate. This glucose isomerization reaction pattern is particularly preferred for producing the maximum effect of the invention, but the present invention is not restricted to such a reaction pattern.

As for the immobilized glucose isomerase used in this invention, there may be employed various known types; for example, a preparation formed by immobilizing an enzyme-containing fungus body or an extracted enzyme on an insolubilized material such as quaternary vinylpyridine resin, DEAE-cellulose, porous alumina, polyphenolic anion exchange resin, styrene-divinylbenzene-based macro-reticular or porous anion exchange resin, anion exchange fibres, and fibres having a specific group (Japanese Patent Laid-Open 6774/75, U.S. Patent 3788945, Japanese Patent Laid-Open 110889/74, 80160/74, 53582/75, 15019/79 and 98912/80), a preparation formed by crosslinking an enzyme-containing fungus body with glutaraldehyde (Japanese Patent Laid-Open 9227/74), and a preparation obtained by incorporating an extracted enzyme in cellulose acetate and spinning the mixture into fibres (Japanese Patent Laid-Open 82084/73). Among them, the preparation formed by immobilizing an extracted enzyme on fibres is particularly preferred because of high enzyme activity.

The present invention is further described hereinbelow by way of the embodiments thereof, but these embodiments are merely intended to be illustrative and not restrictive to the scope of the invention.

### Example 1

By using polypropylene (Mitsui Noblen J—3—HG) as an island component and a mixture of 51.7 parts of polystyrene (Styron 679), 1.5 parts of low-molecular polystyrene (Hymer ST—120), 5.3 parts of polypropylene (Mitsui Noblen WF—727—D) and 0.6 parts of low-molecular weight polypropylene (Viskole 550-P) as a sea component, said sea and island components were mixed at the sea: island ratio of 59.1: 40.9 and subjected to melt composite spinning (the number of islands: 18) at 255°C and the resulting filaments were stretched 5 times the original length to obtain multicore sea-and-island type composite fibres (single filament size: 1.9 d, tenacity: 2.5 g/d). 1.0 part of said fibres moulded into the form of a braid was added into a solution composed of 1.0 part of methylolacrylamide, 10 parts of sulphuric acid, 10 parts of nitrobenzene and 0.03 parts of paraformaldehyde and reacted at room temperature for 4 hours, and the reaction product was extracted with methanol and an acrylamidomethyl group was introduced to the polystyrene portion simultaneously with crosslinking. Then the product was further treated in 20% diethylenetriamine-methanol under reflux for 6 hours to introduce a polyamino group. After conversion into a free type, the product was added to 100 parts of a 50 mM/1 ferric chloride solution and reacted under stirring at room temperature for 4 hours, and the reaction product was washed with water and further treated with a phosphate buffer solution of pH 7 and then with a sodium hydrogen carbonate buffer solution of pH 8.2 to obtain 2.2 parts of an enzyme deactivation inhibitor. The iron content was 0.89 meq/g.

An immoblized preparation of glucose isomerase (available from Novo Inc. of Denmark under the trade name "Sweetzyme-Q") was packed in a column of 1.6 cm inner diameter immersed in a thermostatic tank and said enzyme deactivation inhibitor was further packed in the upper layer portion thereof, and a substrate solution (pH 8.2) composed of an industrial refined dextrose solution containing 5 mM/1 of magnesium sulphate and 3 M/1 of glucose was passed through said column from its top continuously for 30 days at a temperature of 61°C and at a flow rate of approximately 20 ml/hr, and the half-life of the enzyme was determined. The results are shown in Table 1. The half-life of the enzyme was determined according to the method shown in Japanese Patent Publication No. 12238/80, that is, the activity index was determined every other day or on every third day, and since the activity index lowers as an exponential function of the number of days of passing of the solution due to deactivation of the enzyme, it was calculated in terms of the days in which the activity index became half (50%) of the original figure by using the method of least squares.

### Example 2

An acrylamidomethyl group was introduced to 1.0 part of the above-said braid-like moulded multi-core sea-and-island type composite fibres according to the method of Example 1. Then the reaction product was further treated in 20% pentaethylenehexamine-methanol under reflux for 6 hours to introduce a polyamino group. After conversion into a free type, the product was added to 100 parts of a 50 mM/1 aqueous solution of ferric chloride and reacted under stirring at room temperature for 4 hours, and this reaction product was washed with water and then treated with a tris-sulphate buffer solution of pH 8.0 to obtain 2.4 parts of an enzyme deactivation inhibitor. The iron content was 0.86 meq/g. By using the thus obtained inhibitor, a substrate solution was passed through a column in the same manner as in Example 1 except for continuous 7-day passing of the solution at 66°C to obtain a fructose-containing syrup, and the half-life of the enzyme was determined according to the method of Example 1. The results are shown in Table 1.

### Example 3

After introducing an polyamino group to 1.0 part of said braid-like moulded multi-core sea-and-island type composite fibres according to the method of Example 1, the reaction product was further treated with a solution consisting of 6 parts of monochloroacetic acid, 3.5 parts of potassium hydroxide, 6 parts of potassium carbonate, 30 parts of methanol and 10 parts of water under reflux for 6 hours to introduce a polyaminopolyacetic acid group. After additional treatment with an acetate buffer of pH 5, the product was reacted with an iron salt and treated with buffer solutions in the same way as in Example 1 to obtain 2.4 parts of an enzyme deactivation inhibitor. The iron content was 0.94 meq/g. The half-life of the enzyme was determined according to the same method as in Example 2 except for use of said inhibitor instead of the inhibitor obtained in Example 2. The results are shown in Table 1.

### Example 4

10 ml of a commercial chelate reasin having an imino-diacetic acid group (trade name: Diaion CR—10) was buffered with sodium hydrogen carbonate of pH 8.2 and the product was reacted with an iron salt for 20 hours and further treated with buffer solution in the same way as in Example 1 to obtain 2.4 parts of an enzyme deactivation inhibitor. The iron content was 1.43 meq/g. By using the thus obtained inhibitor, the half-life of the enzyme was determined according to the method of Example 2. The results are shown in Table 1.

## Comparative Example 1

The half-life of the enzyme was determined without the enzyme deactivation inhibitor under the conditions of Example 1. The results are shown in Table 1.

## Comparative Example 2

The half-life of the enzyme was determined without the enzyme deactivation inhibitor but 0.1 mM/1 of iron sulphate was added to the substrate solution in Example 1. The results are shown in Table 1. Addition of iron sulphate caused colouration of the substrate solution and also a sediment was formed in the column as the number of days passing the solution increased, thus inhibiting flow of the solution.

## Comparative Example 3

The half-life of the enzyme was determined without the enzymne deactivation inhibitor used in Example 2. The results are shown in Table 1.

It is apparent from Table 1 that the life of insolubilized isomerase is drastically prolonged by performing the enzyme reaction after contacting the substrate solution with a small quantity of the enzyme deactivation inhibitor.

TABLE 1

| | Enzyme deactivation inhibitor | | | | Amount of immobilized enzyme preparation used (g) | Reaction temperature (°C) | Half-life of enzyme (days) |
|---|---|---|---|---|---|---|---|
| | Metal ion | Group bonded to metal ions | Form | Amount used (g) | | | |
| Example 1 | Iron | $-NH(C_2H_4NH)_2H$ | Fiber | 1.0 | | | 56 |
| Comparative Example 1 | No inhibitor used | | | | 4.0 | 61 | 32 |
| Comparative Example 2 | No inhibitor used, but 0.1 mM/1 of iron sulfate was added to the substrate solution | | | | | | 40 |
| Example 2 | | $-NH(C_2H_4NH)_5H$ | Fiber | 0.28 | | | 31 |
| Example 3 | Iron | Polyaminopolyacetic acid group | | | 2.8 | 66 | 25 |
| Example 4 | | Iminodiacetic acid group | Resin | 0.38 | | | 31 |
| Comparative Example 3 | No inhibitor used | | | | | | 16 |

## Claims

1. An isomerization reaction method in which a glucose-containing substrate solution is contacted with a glucose isomerase to produce a fructose-containing syrup characterised in that, prior to the isomerization, the said solution is brought into contact with a synthetic organic ion exchanger to which ions of iron are bonded.

2. A method according to claim 1, wherein said ion exchanger is styrene based.

3. A method according to claim 1 or claim 2 wherein said ion exchanger has ion exchange groups chosen from one or more of the following, viz: amino groups, carboxylic acid groups, phosphoric acid groups, sulphonic acid groups, hydroxyl group, carboxyl group, polycarboxylic acid groups, aldehyde groups, polyamino groups, amino carboxylic acid groups, peptide groups and β-diketone groups.

4. A method according to claim 3 wherein the ion exchange groups are chosen from amino groups, carboxylic acid groups, phosphoric acid groups, sulphonic acid groups, polyamino groups, and amino carboxylic acid groups.

5. A method according to claim 4 wherien the ion exchange groups are —NH(C$_2$H$_4$NH)$_n$H groups (where n is a number of 1 or greater) or —NH(CH$_2$)$_m$NH$_2$ groups (where m is a number of 3 to 12), or a mixture thereof.

6. A method according to claim 4, wherein the ion exchange groups are iminodiacetic acid groups or polyaminopolyacetic acid groups or a mixture thereof.

7. A method according to any preceding claim, wherein the amount of iron is at least 0.01 meq/g.

8. A method according to claim 7, wherein the amount of iron is more than 0.1 meq/g.

9. A method according to claim 8, wherein the amount of iron is within the range from 0.5 to 2.5 meq/g.

10. A method according to any preceding claim, wherein the ion exchanger is in the form of powder, fine grain, resinous grain, fibres, hollow fibres, film, paper or a mixture of any of these.

11. A method according to claim 10, wherein the ion exchanger is in the form of macroreticular or porous resinous grain.

12. A method according to claim 10, wherein the ion exchanger is in the form of core-sheath type composite fibres.

13. A method according to claim 12, wherein said core component is made from at least one polymer chosen from polyamides, polyesters, poly-α-olefins, po lyacrylonitriles and copolymers thereof.

14. A method according to any preceding claim, wherein said glucose isomerase is in the form of immobilized glucose isomerase.

15. A method according to claim 14, wherein said immobilized glucose isomerase is a preparation formed by immobilizing an isomerase-containing fungus body or extracted isomerase on an insolubilized material or a preparation formed by crosslinking isomerase-containing fungus body with glutaraldehyde or a prepartion obtained by incorporating extracted isomerase in cellulose acetate and spinning the mixture into fibre or a mixture thereof.

16. A method according to claim 15, wherein said insolubilized material is quaternary vinylpyridine resin, DEAE-cellulose, porous alumina, polyphenolic anion exchange resin, styrene-divinylbenzene-based macro-reticular or porous anion exchange resin, anion exchange fibres, or a mixture thereof.

17. A method according to any one of claims 14 to 16, wherein 30 to 60% by weight of a glucose solution (pH 6.5—9.0) containing 0.1—20 mM/1 of magnesium ions is passed through a single or several fixed beds composed of first-stage layers packed with a synthetic organic ion exchanger to which ions of iron are bonded and second-stage layers of said immobilized glucose isomerase at a temperature of 55—70°C.

## Patentansprüche

1. Isomerisierungsverfahren, bie dem eine Glukose-haltige Substratlösung mit einer Glukose-Isomerase in Berührung gebracht wird, um einen Fruktose-haltigen Sirup zu erzeugen, dadurch gekennzeichnet, daß vor der Isomerisierung die genannte Lösung mit einem synthetischen organischen Ionenaustauscher in Berührung gebracht wird, an den Eisenionen gebunden sind.

2. Verfahren nach Anspruch 1, bei dem der genannte Ionenaustauscher auf Styrolbasis beruht.

3. Verfahren nach Anspruch 1 oder 2, bei dem der genannte Ionenaustauscher ionenaustauschende Gruppen aufweist, die aus einer oder mehreren der folgenden Gruppen ausgewählt sind: Amino-, Karbonsäure-, Phosphorsäure-, Sulfonsäure-, Hydroxyl-, Karboxyl-, Polykarbonsäure-, Aldehyd-, Polyamino-, Aminokarbonsäure-, Peptid- und β-Diketongruppen.

4. Verfahren nach Anspruch 3, bei dem die ionenaustauschenden Gruppen ausgewählt sind aus Amino-, Karbonsäure-, Phosphorsäure-, Sulfonsäure-, Polyamino- und Aminokarbonsäuregruppen.

5. Verfahren nach Anspruch 4, bei dem die ionenaustauschenden Gruppen —NH(C$_2$H$_4$NH)$_n$H-Gruppen (worin n eine Zahl von 1 oder mehr ist) oder —NH(CH$_2$)$_m$NH$_2$-Gruppen (worin m eine Zahl von 3 bis 12 ist) oder eine Mischung davon sind.

6. Verfahren nach Anspruch 4, bei dem die ionenaustauschenden Gruppen Iminodiessigsäure- oder Polyaminopolyessigsäuregruppen oder eine Mischung davon sind.

7. Verfahren nach irgendeinem vorhergehenden Anspruch, bei dem die Eisenmenge mindestens 0,01 mval/g beträgt.

8. Verfahren nach Anspruch 7, bei dem die Eisenmenge mehr als 0,1 mval/g beträgt.

9. Verfahren nach Anspruch 8, bei dem die Eisenmenge im Bereich von 0,5 bis 2,5 mval/g liegt.

10. Verfahren nach irgendeinem vorhergehenden Anspruch, bei dem der Ionenaustauscher in Form von Pulver, als feines Korn, harzartiges Korn, Fasern, Hohlfasern, Film, Papier oder als Mischung irgendwelcher der genannten Formen vorliegt.

11. Verfahren nach Anspruch 10, bei dem der Ionenaustauscher als grob netzförmiges oder poröses harzartiges Korn vorliegt.

12. Verfahren nach Anspruch 10, bei dem der Ionenaustauscher die Form von Verbundfasern aus kern und Hülle hat.

13. Verfahren nach Anspruch 12, bei dem der Kern hergestellt ist aus mindestens einem Polymer ausgewählt aus Polyamiden, Polyestern, Poly-α-olefinen, Polyacrylnitrilen und deren Copolymeren.

14. Verfahren nach irgendeinem vorhergehenden Anspruch, bei dem die genannte Glukose-Isomerase in Form immobilisierter Glukose-Isomerase vorliegt.

15. Verfahren nach Anspruch 14, bei dem die genannte immobilisierte Glukose-Isomerase eine Zubereitung ist, die gebildet ist durch Immobilisieren eines Isomerase-haltigen Pilzkörpers oder von extrahierter Isomerase auf einem unlöslich gemachten Material oder es ist eine Zubereitung, die gebildet ist durch Vernetzen von Isomerasehaltigem Pilzkörper mit Glutaraldehyd oder es ist eine Zubereitung, die erhalten ist durch Einbringen von extrahierter Isomerase in Zelluloseacetat und Spinnen der Mischung zu Fasern oder eine Mischung der genannten Zubereitungen.

16. Verfahren nach Anspruch 15, bei dem das unlöslich gemachte Material quarternäres Vinylpyridinharz, DEAE-Zellulose, poröses Aluminiumoxid, polyphenolisches Anionenaustauscherharz, grob netzförmiges oder poröses Anionenaustauscherharz auf Styrol-Divinylbenzol-Grundlage, Anionenaustauscherfasern oder eine Mischung davon ist.

17. Verfahren nach irgendeinem der Ansprüche 14 bis 16, bei dem bei einer Temperatur von 55—70°C 30 bis 60 Gew.-% einer Glukoselösung (pH 6,5—9,0), die 0,1—20 mM/1 an Magnesiumionen enthält, durch ein einzelnes oder mehrere Festbetten geleitet wird, die zusammengesetzt sind aus Schichten der ersten Stufe, die gepackt sind mit einem synthetischen organischen Ionenaustauscher, an den Eisenionen gebunden sind und aus Schichten der zweiten Stufe aus der genannten immobilisierten Glukose-Isomerase.

**Revendications**

1. Procédé de réaction d'isomérisation dans lequel une solution de substrat contenant du glucose est mise en contact avec une glucose isomérase pour produire un sirop contenant du fructose, caractérisé en ce qu'avant l'isomérisation, cette solution est mise en contact avec un échangeur d'ions organique synthétique auquel des ions fer sont liés.

2. Procédé selon la revendication 1, dans lequel cet échangeur d'ions est à la base de styrène.

3. Procédé selon la revendication 1 ou la revendication 2, dans lequel cet échangeur d'ions a des groupes échangeurs d'ions choisis parmi un ou plusieurs des suivants: à savoir: les groupes amino, les groupes acide carboxylique, les groupes acide phosphorique, les groupes acide sulfonique, les groupes hydroxyle, les groupes carboxyle, les groupes acide polycarboxylique, les groupes aldéhyde, les groupes polylamino, les groupes acid aminocarboxylique, les groupes peptide et les groupes β-dicétone.

4. Procédé selon la revendication 3, dans lequel les groupes échangeurs d'ions sont choisis parmi les groupes amino, les groupes acide carboxylique, les groupes acide phosphorique, les groupes acide sulfonique, les groupes polyamino, et les groupes acide aminocarboxylique.

5. Procédé selon la revendication 4, dans lequel les groupes échangeurs d'ions sont des groupes —NH(C$_2$H$_4$NH)$_n$H (où n est un nombre égal à 1 ou davantage) ou des groupes —NH(CH$_2$)$_m$ NH$_2$ (où m est un nombre de 3 à 12), ou un mélange de ceux-ci.

6. Procédé selon la revendication 4, dans lequel les groupes échangeurs d'ions sont des groupes acide iminodiacétique ou des groupes acide polyaminopolyacétique ou un mélange de ceux-ci.

7. Procédé selon l'une quelconque des revendications precédentes, dans lequel la quantité de fer est d'au moins, 0,01 meq/g.

8. Procédé selon la revendication 7, dans lequel la quantité de fer est supérieure à 0,1 meq/g.

9. Procédé selon la revendication 8, dans lequel la quantité de fer est dans l'intervalle de 0,5 à 2,5 meq/g.

10. Procédé selon l'une quelconque des revendications précédentes, dans lequel l'échangeur d'ions est sous la forme de poudre, de grain fin, de grain résineux, de fibres, de fibres creuses, de pellicule, de papier ou d'un mélange de n'importe lesquels de ceux-ci.

11. Procédé selon la revendication 10, dans lequel l'échangeur d'ions est sous la forme d'un grain résineux macro-réticulaire ou poreux.

12. Procédé selon la revendication 10, dans lequel l'échangeur d'ions est sous la forme de fibres composites du type âme-enveloppe.

13. Procédé selon la revendication 12, dans lequel ce constituant d'âme est constitué d'au moins un polymère choisi parmi les polyamides, les polyesters, les poly-α-oléfines, les polyacrylonitriles et des copolymères de ceux-ci.

14. Procédé selon l'une quenconque des revendications précédentes, dans lequel cette glucose isomérase est sous la forme d'une glucose isomérase immobilisée.

15. Procédé selon la revendication 14, dans lequel cette glucose isomérase immobilisée est une préparation formée en immobilisant un organisme fongique contenant de l'isomérase ou de l'isomérase extraite sur une matière insolubilisée ou une préparation formée en réticulant un organisme fongique contenant de l'isomérase avec du glutaraldéhyde ou une préparation obtenue en incorporant de l'isomérase extraite à de l'acétate de cellulose et en filant le mélange en fibre, ou un mélange ce celles-ci.

16. Procédé selon la revendication 15, dans lequel cette matière insolubilisée est une résine de vinylpyridine quaternaire, de la DEAE-cellulose, de l'alumine poreuse, une resine échangeuse d'anions polyphénolique, une résine échangeuse d'anions macro-réticulaire ou poreuse à base de styrène-divinylbenzène, des fibres échangeuses d'anions ou un mélange de celles-ci.

17. Procédé selon l'une quelconque des revendications 14 à 16, dans lequel 30 à 60% en poids d'une solution de glucose (pH 6,5—9,0) contenant 0,1—20 mM/1 d'ions magnésium est envoyée à travers un seul ou plusieurs lits fixes composés de couches du premier stade garnies d'un échangeur d'ions organique synthétique auquel sont liés des ions fer, et des couches de second stade de cette glucose-isomérase immobilisée à une température de 55—70°C.